# EUROPEAN PATENT APPLICATION

(11) **EP 1 548 127 A1**
(43) Date of publication of application: **29.06.2005**
(21) Application number: 03791336.5
(22) Date of filing: 27.08.2003
(51) Int. Cl.: C12Q 1/68, C12N 15/31

(54) **PRIMER AND PROBE FOR DETECTING i VIBRIO VULNIFICUS /i AND DETECTION METHOD USING THE SAME**

(30) Priority: 30.08.2002 JP 2002255126
(71) Applicant: Nichirei Corporation, Tokyo 104-8402 (JP)
(72) Inventor: KOIZUMI, T., R & D Division, Nichirei Corporation, Chiba-shi, Chiba 261-8545 (JP); NISHIYAMA, Y., R & D Div., Nichirei Corporation, Chiba-shi, Chiba 261-8545 (JP); YAMAMOTO, S., R & D Division, Nichirei Corporation, Chiba-shi, Chiba 261-8545 (JP); FUKUYAMA, M., College of Environmental Health, Sagamihara-shi, Kanagawa 229-8501 (JP); FURUHATA, K., College of Environmental Health, Sagamihara-shi, Kanagawa 229-8501 (JP); OONAKA, Kenji, College of Environmental Health, Sagamihara-shi, Kanagawa 229-8501 (JP)
(74) Representative: Woods, Geoffrey Corlett
(86) International application number: PCT/JP2003/010846
(87) International publication number: WO 2004/020671

(57) **Abstract**

To produce a high-performance specific gene amplification primer for detecting, quantifying, or identifying *Vibrio vulnificus*, having low risk of misidentification and practically sufficient amplification efficiency and amplification specificity. We have determined partial nucleotide sequences of *gyrB, rpoD*, and *recA* genes of *Vibrio vulnificus* and the closely related species, revealed their phylogenetic relationship, and then identified nucleotides characteristic to *Vibrio vulnificus*. Thus, we have made it possible to design a probe having high specificity and a gene amplification primer having high specificity and excellent amplification efficiency, both of which contain the characteristic nucleotides.

SEQUENCE LISTING

<110> Nichirei Corporation

<120> Method of the detection of Vibrio vulinificus and primers and probes
therefor

<130> PH-1839-PCT

<150> JP 2002-255126
<151> 2002-08-30

<160> 20

<170> PatentIn Ver. 2.1

<210> 1
<211> 885
<212> DNA
<213>

<400> 1
gtgtctggcg gtcttcacgg tgtwggygtt tcggtggtra acgcaytgtc tgaaaaagtr 60
ctrytkacga ttcatcgtgg tggtcatacy cayagccaaa cctatcgtca tggtgtgcct 120
gatgcaccgc ttgctatcat cggygatacy gaaaaaaccg gtaccacggt acgtttctgg 180
ccaagtgcks aaaccttcas caacatcgaa tttcattatg acatcctagc gaagcgtyta 240
cgtgagctct ctttyctgaa ctckggcgtg tckatcaaac tgtttgatga gcgcgaagaa 300
gataagraag atcacttcat gtatgaaggt ggtattcaag cgttygtyac tcacttgaac 360
cgcaacaara cmcccatcca tgaaaaagta ttccatttya aykcygagcg tgaagacggk 420

attgckgttg aagtggcgat gcagtggaac gatggyttcc aagaaaacat ctactgtttt 480
accaacaaca tcccacagcg tgayggtggt acccacttag cgggtttccg ygcggcattg 540
acgcgcacay tgaacagcta catggacaaa gaaggytact craagaaagc gaaaaccgcg 600
acttctggyg aygatgcgcg tgaaggtttg acygcwgtyg tttcagthaa agtrccggat 660
ccaaaattct caagccaaac yaaagacaaa ctggtttcta gygaagtraa gtccgcagtg 720
gaatccttca tggcagacaa actgaacgac ttcttrgcyg arcacccaag cgaagcgaaa 780
accgtttgtt ctaagattat cgacgccgca cgtgcgcgtg aagcagcrcg taaagcgcgt 840
gaaatgacrc gccgtaaagg ggcrttagay cttgctggcc ttccw 885

<210> 2
<211> 819
<212> DNA
<213>

<400> 2
actcgtgaag gcgaaatcga catcgctaam cgaattgaag atggtatcaa ccaagtacag 60
tcstctgttg ctgaataccc aggaaccatt ccttatattc tkgaacagtt cgacaaagta 120
caagcggaag aacttcgcct mactgatctt atcagtggtt ttgttgatcc aaatgcagat 180
gaaacggcwg ctccaaccgc aacacacaty ggttcagagc ttgcagaatc tgatttggaa 240
gatgaagaca ayaccgacat cgacgatgaa gacgaagayn nnnnngaaga tggcgattca 300
agcagygatt cagaggamga tgtcggcatc gaccctgaaa tggcgctaga gaagttyact 360
carcttcgta acagctayca gaatctgcaa cttgccktra atgaacatgg tcgagagagt 420
gctcaaacag ctcaagccca tgaactgatg ctcgatgtgt ttaaagagtt tcgtctaacd 480
ccgaagcagt ttgaccattt ggttaacgaa cttcgcaccg cyatggatcg cgttcgtack 540
caagarcgyt tgatcatgaa rtctgcggta gaaatcgcsa aratgccraa gaartcktty 600
atygcwctct tyactggcaa cgartcwarc gaagaatggt tagatmagat cctmgyytct 660
gayaagccrt acgyagaaaa gatyaarctk cacgaagaag acattcgtcg ttcaatcdcc 720
aagctaagag caattgaaga agaaacgtcg ctttcagtra rcaacatcaa agacatcagc 780

cgtcgtatgt ctatmggtga agcmaaagct cgccgtgcg 819

<210> 3
<211> 648
<212> DNA
<213>

<400> 3
ccaatgggcc gtatcgttga aatttttggt ccagaatctt caggtaaaac cacgttgacc 60
cttgagctga tcgctgcrgc tcaacgtgaa ggcaaaactt gtgcgtttat cgatgcygag 120
cacgcgttrg atcctgtgta tgcgaagaar cttggcgtwa atatcgacca rttrttggta 180
tctcagccyg ayacbggtga acaagcrttg gaaatctgtg atgckcttgc tcgctcaggk 240
gcggttgayg ttattgttgt cgaytctgtk gcmgcattga crccaaaggc agaaatygaa 300
ggtgagatgg gygaytcgca catgggtctn caagctcgta tgctmtctca agcgatgcgt 360
aagytaacgg gkaacctaaa rcagtctaac tgtatgtgta tcttcatyaa ccagatycgt 420
atgaagatyg gkgtgatgtt tggtaaycca gaaaccacaa crggtggtaa cgcwctgaaa 480
ttctacgctt ctgtwcgtct tgatattcgc cgtactggtg cratcaaaga aggygatgag 540
gtmgtgggta aygaaacgcg yatcaaagtg gtgaagaata agatcgctgc gccgtttaaa 600
gaagccaaya cycaaattat gtayggccar ggcwttaacc gygaaggy 648

<210> 4
<211> 15
<212> DNA
<213> Artificial Sequence

<220>
<223> Description of Artificial Sequence:primer

<400> 4

gtgtctggcg gtctt                                                        15

<210> 5

<211> 21

<212> DNA

<213> Artificial Sequence

<220>

<223> Description of Artificial Sequence:primer

<400> 5

gatgcaccgc ttgctatcat c                                                 21

<210> 6

<211> 18

<212> DNA

<213> Artificial Sequence

<220>

<223> Description of Artificial Sequence:primer

<400> 6

tgcggactty acttcrct                                                     18

<210> 7

<211> 19

<212> DNA

<213> Artificial Sequence

<220>

<223> Description of Artificial Sequence:primer

<400> 7

gtccatgtag ctgttcart 19

<210> 8

<211> 21

<212> DNA

<213> Artificial Sequence

<220>

<223> Description of Artificial Sequence:primer

<400> 8

ttgtctgcca tgaaggattc c 21

<210> 9

<211> 19

<212> DNA

<213> Artificial Sequence

<220>

<223> Description of Artificial Sequence:primer

<400> 9
aatcgacatc gctaamcga                                                    19

<210> 10
<211> 19
<212> DNA
<213> Artificial Sequence

<220>
<223> Description of Artificial Sequence:primer

<400> 10
gttcgacaaa gtacaagcg                                                    19

<210> 11
<211> 18
<212> DNA
<213> Artificial Sequence
<220>
<223> Description of Artificial Sequence:primer

<400> 11
tcaagcagyg attcagag                                                     18

<210> 12

<211> 16

<212> DNA

<213> Artificial Sequence

<220>

<223> Description of Artificial Sequence:primer

<400> 12

aatggcgcta gagaag                                                    16

<210> 13

<211> 19

<212> DNA

<213> Artificial Sequence

<220>

<223> Description of Artificial Sequence:primer

<400> 13

cktraatgaa catggtcga                                                 19

<210> 14

<211> 21

<212> DNA

<213> Artificial Sequence

<220>

<223> Description of Artificial Sequence:primer

<400> 14
gaactgatgc tcgatgtgtt t                                                21

<210> 15
<211> 20
<212> DNA
<213> Artificial Sequence

<220>
<223> Description of Artificial Sequence:primer

<400> 15
aatgtcttct tcgtgmagyt                                                  20

<210> 16
<211> 21
<212> DNA
<213> Artificial Sequence

<220>
<223> Description of Artificial Sequence:primer

<400> 16
ttgatgttgy tyactgaaag c                                                21

<210> 17

<211> 21

<212> DNA

<213> Artificial Sequence

<220>

<223> Description of Artificial Sequence:primer

<400> 17

cctgtgtatg cgaagaarct t                                                21

<210> 18

<211> 19

<212> DNA

<213> Artificial Sequence

<220>

<223> Description of Artificial Sequence:primer

<400> 18

tatcgaccar ttrttggta                                                   19

<210> 19

<211> 21

<212> DNA

<213> Artificial Sequence

<220>

<223> Description of Artificial Sequence:primer

<400> 19

aagmgcatca cagatttcca a                                                21

<210> 20

<211> 21

<212> DNA

<213> Artificial Sequence

<220>

<223> Description of Artificial Sequence:primer

<400> 20

tcaaccgcmc ctgagcgagc a                                                21

SEQUENCE LISTING

<110> Nichirei Corporation

<120> Primer and probe for detecting Vibrio vulnificus and detection
method using the same

<130> N.94227 GCW

<140> EP 03791336.5
<141> 2003-08-27

<150> PCT/JP03/10846
<151> 2003-08-27

<150> JP 2002-255126
<151> 2002-08-30

<160> 20

<170> PatentIn Ver. 2.1

<210> 1
<211> 885
<212> DNA
<213> Vibrio vulnificus

<400> 1
gtgtctggcg gtcttcacgg tgtwggygtt tcggtggtra acgcaytgtc tgaaaaagtr 60
ctrytkacga ttcatcgtgg tggtcatacy cayagccaaa cctatcgtca tggtgtgcct 120
gatgcaccgc ttgctatcat cggygatacy gaaaaaaccg gtaccacggt acgtttctgg 180
ccaagtgcks aaaccttcas caacatcgaa tttcattatg acatcctagc gaagcgtyta 240
cgtgagctct ctttyctgaa ctckggcgtg tckatcaaac tgtttgatga gcgcgaagaa 300
gataagraag atcacttcat gtatgaaggt ggtattcaag cgttygtyac tcacttgaac 360
cgcaacaara cmcccatcca tgaaaaagta ttccatttya aykcygagcg tgaagacggk 420
attgckgttg aagtggcgat gcagtggaac gatggyttcc aagaaaacat ctactgtttt 480
accaacaaca tcccacagcg tgayggtggt acccacttag cgggtttccg ygcggcattg 540
acgcgcacay tgaacagcta catggacaaa gaaggytact craagaaagc gaaaaccgcg 600
acttctggyg aygatgcgcg tgaaggtttg acygcwgtyg tttcagthaa agtrccggat 660
ccaaaattct caagccaaac yaaagacaaa ctggtttcta gygaagtraa gtccgcagtg 720
gaatccttca tggcagacaa actgaacgac ttcttrgcyg arcacccaag cgaagcgaaa 780
accgtttgtt ctaagattat cgacgccgca cgtgcgcgtg aagcagcrcg taaagcgcgt 840
gaaatgacrc gccgtaaagg ggcrttagay cttgctggcc ttccw 885

<210> 2
<211> 819
<212> DNA
<213> Vibrio vulnificus

<400> 2
actcgtgaag gcgaaatcga catcgctaam cgaattgaag atggtatcaa ccaagtacag 60
tcstctgttg ctgaataccc aggaaccatt ccttatattc tkgaacagtt cgacaaagta 120
caagcggaag aacttcgcct mactgatctt atcagtggtt ttgttgatcc aaatgcagat 180
gaaacggcwg ctccaaccgc aacacacaty ggttcagagc ttgcagaatc tgatttggaa 240
gatgaagaca ayaccgacat cgacgatgaa gacgaagayn nnnnngaaga tggcgattca 300
agcagygatt cagaggamga tgtcggcatc gaccctgaaa tggcgctaga gaagttyact 360
carcttcgta acagctayca gaatctgcaa cttgccktra atgaacatgg tcgagagagt 420
gctcaaacag ctcaagccca tgaactgatg ctcgatgtgt ttaaagagtt tcgtctaacd 480
ccgaagcagt ttgaccattt ggttaacgaa cttcgcaccg cyatggatcg cgttcgtack 540
caagarcgyt tgatcatgaa rtctgcggta gaaatcgcsa aratgccraa gaartcktty 600

atygcwctct tyactggcaa cgartcwarc gaagaatggt tagatmagat cctmgyytct 660
gayaagccrt acgyagaaaa gatyaarctk cacgaagaag acattcgtcg ttcaatcdcc 720
aagctaagag caattgaaga agaaacgtcg ctttcagtra rcaacatcaa agacatcagc 780
cgtcgtatgt ctatmggtga agcmaaagct cgccgtgcg 819

<210> 3
<211> 648
<212> DNA
<213> Vibrio vulnificus

<400> 3
ccaatgggcc gtatcgttga aatttttggt ccagaatctt caggtaaaac cacgttgacc 60
cttgagctga tcgctgcrgc tcaacgtgaa ggcaaaactt gtgcgtttat cgatgcygag 120
cacgcgttrg atcctgtgta tgcgaagaar cttggcgtwa atatcgacca rttrttggta 180
tctcagccyg ayacbggtga acaagcrttg gaaatctgtg atgckcttgc tcgctcaggk 240
gcggttgayg ttattgttgt cgaytctgtk gcmgcattga crccaaaggc agaaatygaa 300
ggtgagatgg gygaytcgca catgggtctn caagctcgta tgctmtctca agcgatgcgt 360
aagytaacgg gkaacctaaa rcagtctaac tgtatgtgta tcttcatyaa ccagatycgt 420
atgaagatyg gkgtgatgtt tggtaaycca gaaaccacaa crggtggtaa cgcwctgaaa 480
ttctacgctt ctgtwcgtct tgatattcgc cgtactggtg cratcaaaga aggygatgag 540
gtmgtgggta aygaaacgcg yatcaaagtg gtgaagaata agatcgctgc gccgtttaaa 600
gaagccaaya cycaaattat gtayggccar ggcwttaacc gygaaggy 648

<210> 4
<211> 15
<212> DNA
<213> Artificial Sequence

<220>
<223> Description of Artificial Sequence:primer

<400> 4
gtgtctggcg gtctt 15

<210> 5
<211> 21
<212> DNA
<213> Artificial Sequence

<220>
<223> Description of Artificial Sequence:primer

<400> 5
gatgcaccgc ttgctatcat c 21

<210> 6
<211> 18
<212> DNA
<213> Artificial Sequence

<220>
<223> Description of Artificial Sequence:primer

<400> 6
tgcggactty acttcrct 18

<210> 7
<211> 19
<212> DNA
<213> Artificial Sequence

<220>
<223> Description of Artificial Sequence:primer

<400> 7
gtccatgtag ctgttcart                                                    19


<210> 8
<211> 21
<212> DNA
<213> Artificial Sequence

<220>
<223> Description of Artificial Sequence:primer

<400> 8
ttgtctgcca tgaaggattc c                                                 21

<210> 9
<211> 19
<212> DNA
<213> Artificial Sequence

<220>
<223> Description of Artificial Sequence:primer

<400> 9
aatcgacatc gctaamcga                                                    19


<210> 10
<211> 19
<212> DNA
<213> Artificial Sequence

<220>
<223> Description of Artificial Sequence:primer

<400> 10
gttcgacaaa gtacaagcg                                                    19


<210> 11
<211> 18
<212> DNA
<213> Artificial Sequence
<220>
<223> Description of Artificial Sequence:primer

<400> 11
tcaagcagyg attcagag                                                     18


<210> 12
<211> 16
<212> DNA

<213> Artificial Sequence

<220>
<223> Description of Artificial Sequence:primer

<400> 12
aatggcgcta gagaag                                                    16


<210> 13
<211> 19
<212> DNA
<213> Artificial Sequence

<220>
<223> Description of Artificial Sequence:primer

<400> 13
cktraatgaa catggtcga                                                 19


<210> 14
<211> 21
<212> DNA
<213> Artificial Sequence

<220>
<223> Description of Artificial Sequence:primer

<400> 14
gaactgatgc tcgatgtgtt t                                              21


<210> 15
<211> 20
<212> DNA
<213> Artificial Sequence

<220>
<223> Description of Artificial Sequence:primer

<400> 15
aatgtcttct tcgtgmagyt                                                20


<210> 16
<211> 21
<212> DNA
<213> Artificial Sequence

<220>
<223> Description of Artificial Sequence:primer

<400> 16
ttgatgttgy tyactgaaag c                                              21


<210> 17
<211> 21
<212> DNA
<213> Artificial Sequence

<220>
<223> Description of Artificial Sequence:primer

<400> 17
cctgtgtatg cgaagaarct t                                                21


<210> 18
<211> 19
<212> DNA
<213> Artificial Sequence

<220>
<223> Description of Artificial Sequence:primer
<400> 18
tatcgaccar ttrttggta                                                   19


<210> 19
<211> 21
<212> DNA
<213> Artificial Sequence

<220>
<223> Description of Artificial Sequence:primer

<400> 19
aagmgcatca cagatttcca a                                                21


<210> 20
<211> 21
<212> DNA
<213> Artificial Sequence

<220>
<223> Description of Artificial Sequence:primer

<400> 20
tcaaccgcmc ctgagcgagc a                                                21

## Description

### TECHNICAL FIELD

The present invention relates to a method for detecting, identifying, or counting *Vibrio vulnificus* in food inspection, epidemiological environment inspection, and clinical examination.

### BACKGROUND ART

*Vibrio vulnificus* is a halophilic gram-negative bacterium inhabiting seawater and brackish water areas. Infection in a healthy human is rare, but *Vibrio vulnificus* may infect a patient with a severe underlying disease such as liver cirrhosis and may cause serious infectious diseases. It has attracted attention in public health as an infectious bacterium associated with cases of death. The first case report of a infectious disease resulting from this bacterium involved isolation of the bacterium by Roland from gangrene in lower extremities in 1970 (New Engl. J. Med., 282, 1306, 1970). This case was initially thought to be a case of parenteral infection with *Vibrio parahaemolyticus*. It has since been revealed that this bacterium is capable of degrading lactose more slowly than *Escherichia coli* and the like and incapable of proliferating in the presence of 8% NaCl. In 1979, the bacterium was named *Vibrio vulnificus* by Farmer (Lancet, ii, 903, 1979).

This bacterium is classified based on its biological properties into biotypes 1 to 3 (Appl. Environ. Microbiol., 63, 1460-1466, 1997, Lancet, 354, 1421-1424, 2000). Those isolated from human infectious diseases are mainly of type 1, and those of type 2 are mainly isolated as pathogenic bacteria infecting fish. Furthermore, those of type 3 were isolated as pathogenic bacteria causing mass infection in Israel, which occurred during 1996 and 1997 (Lancet, 354, 1421-1424, 2000).

Infection patterns of this bacterium include an oral infection type and a wound infection type. Oral infection is mainly caused by eating of raw seafood in summer, with a complication of sepsis after the development of symptoms of digestive system diseases such as onset of fever, abdominal pain, diarrhea, and/or vomit within 24 hours after infection, leading to death within an average of 48 hours. The mortality rate is very high, between 50% and 70%. Moreover, wound infection is caused by the contact of wounds with seawater, with the onset occurring within 1 week after a 12-hour incubation period. Mortality rate is 25%, which is lower than that of oral infection. In the U.S.A., 422 cases were reported in 22 states from 1988 to 1996. As for the source of infection, most cases resulted from the intake of raw oysters, reflecting an American dietary habit. In the meantime, about 90 cases have been reported on the Pacific side of Kanto and westward in Japan, and infection is mainly caused by eating of raw seafood such as sashimi and sushi. Recently, for example in Kumamoto in July of 2001, 4 people were infected with the bacterium by eating raw squillas and flatheads, and 2 of them died. In Japan, with its culture of eating raw seafood, infectious diseases caused by *Vibrio vulnificus* can be said to be a cause of alarm.

In selective isolation of the bacterium, a TCBS agar medium has been principally used, similarly to that used for *Vibrio parahaemolyticus. Vibrio vulnificus* forms greenish-blue colonies that are very similar to those formed by *Vibrio parahaemolyticus* on a TCBS medium. It is required to confirm various biochemical properties for differentiation. The confirmation process is complicated and involves a danger of misidentification. At the same time, improved selective isolation media such as SPS agar (SDS-polymixin B sucrose agar) media (FEMS Microbiol. Lett., 17, 205-209, 1983) or cellobiose-collistin agar media (Appl. Environment. Microbiol., 64, 1721-1724, 1998) have also been reported, but they are the same in terms of requiring a procedure to confirm biochemical properties.

Meanwhile, some genetic-engineering-technique-applied methods for detecting *Vibrio vulnificus* have been developed, similarly to the cases of other food-poisoning bacteria. "wza" is a gene discovered from *E. coli* as a gene encoding a transporter that is necessary for transporting CPS (capsular polysaccharide) from the periplasm to the outer membrane. In the case of *Vibrio vulnificus*, since a strain lacking capsules does not present any toxicity against mice and a wza-deficient strain loses its pathogenicity against mice (*Infect. Immun*. 69, 6893-6901, 2001), the wza is thought to be one of pathogenicities. Accordingly, as a means for detecting *Vibrio vulnificus* with pathogenicities, a primer targeting the wza gene has been developed by Wright et al. (U. S. Patent 6183973, Feb., 6, 2001). However, the pathogenic factor of *Vibrio vulnificus* is not limited to CPS, so that not all *Vibrio vulnificus* bacteria with pathogenicities can always be detected. Furthermore, a primer targeting a hemolysin gene (*vvh*: *Vibrio vulnificus* hemolysin) that is thought to also be one of pathogenic factors of *Vibrio vulnificus* has been developed (Appl. Environ. Microbiol. 57, 707-711, 1991). This primer has been designed on the assumption that the vvh gene is a gene specific to *Vibrio vulnificus*. However, it has been reported that hemolysin genes of *V. cholerae* and *V. mimicus* show homology with the vvh gene (Infect. Immun. 58, 2706-2709, 1990; Infect. Immun. 65, 1830-1835, 1997). Moreover, upon designing the primer, comparison with these hemolysin genes has not been conducted. Thus, it cannot be said that specificity has been sufficiently verified.

As described above, regarding the existing methods for detecting *Vibrio vulnificus* using genes, it cannot be said that specificity is sufficient.

### SUMMARY OF THE INVENTION

What is common among conventional genetic screening methods is that these methods ignore the fact that a bacterial "species" is a population containing genetic diversity. The use of a nucleotide sequence of 1 strain that is inferred to be a member of a bacterial population as a common sequence of the population or a sequence representing the population is very dangerous in terms of molecular evolutional characteristics of genes rapidly accumulating neutral mutations. Specifically, it is a concern that such a use could cause misidentification such that a strain originally to be detected could not be detected because of inhibited amplification due to a slight mutation in a primer region. It is also a concern that a closely related strain not to be detected would be detected because of insufficient specificity of a primer. Hence, it has been required to produce a high-performance and specific gene amplification primer and a high-performance and specific probe for detecting, identifying, or quantifying *Vibrio vulnificus*, which has a proven background of specificity, low possibility of misidentification, and practically sufficient amplification efficiency and amplification specificity.

To produce a method for specifically detecting a gene of a bacterial phylogenetic group, there is a need to collect as many nucleotide sequences as possible of a biological group to be detected and of biological groups phylogenetically close to these groups. Moreover, a target gene of specific detection should have a sufficiently different nucleotide sequence so that it is distinguishable from the most closely related organisms.
For this, a target gene must have a sufficiently rapid rate of evolution. In addition, as in the case of a gene that is transmitted horizontally at a high frequency(e.g., a toxin gene of *Vibrio parahaemolyticus*), a gene that exists independently from the phylem cannot be used. Proteins encoded by *gyrB* gene, *rpoD* gene, and *recA* gene used as targets in the present invention are essential for survival. Because of this reason, these genes are hardly transmitted horizontally and have appropriate rates of evolution, so that they are preferably used for bacterial phylogenetic analysis. We have already developed a convenient method for determining a nucleotide sequence, which involves subjecting the *gyrB* gene and the *rpoD* gene to the PCR direct sequencing method (JP Patent Publication (Kokai) Nos. 07-213229 A (1995) and 08-256798 A (1996)).

Furthermore, we have already isolated large quantities of *Vibrio vulnificus* from the environment. In the meantime, regarding a known stock strain of *Vibrio vulnificus* and those reported to be related to this bacterium according to analysis based on a 16S rRNA sequence, *V. navarrensis, V. diazotrophicus, V. ordalii, Listonella anguillarum, V. metschnikovii, V. cincinnatiensis, V. mimicus,* and *V. chorelae* (International Journal of Systematic and Evolutionary Microbiology 51, 1449-1456 (2001)), we have analyzed the partial nucleotide sequences of the *gyrB, rpoD*, and *recA* genes of strains listed in the following Table 1 and conducted phylogenetic analysis based on the sequences, thereby revealing the phylogenetic relationship.

**Table 1.**

| Strains used | | | |
|---|---|---|---|
| *V. vulnificus* | 32 strains | ATCC 27562 T | |
| | | ATCC 29306 | |
| | | ATCC 29307 | |
| | | ATCC 33147 | |
| | | ATCC 33148 | |
| | | ATCC 33149 | |
| | | ATCC 33814 | |
| | | ATCC 33815 | |
| | | ATCC 33816 | |
| | | ATCC 33817 | |
| | | ATCC 43382 | |
| | | ATCC BAA-86 | |
| | | ATCC BAA-87 | |
| | | ATCC BAA-88 | |
| | | ATCC BAA-89 | |
| | | ATCC BAA-90 | |
| | | JCM 3726 | |
| | | JCM 3727 | |
| | | JCM 3728 | |
| | | JCM 3729 | |
| | | JCM 3730 | |
| | | JCM 3731 | |
| | | Strains isolated from environment | 10 strains |
| Other stock strains of the genus *Vibrio* | 48 strains | | |
| *V. cholerae* | 29 strains | Clinically isolated strains | 29 strains |
| *V. mimicus* | 4 strains | ATCC 33653 T | |
| | | ATCC 33654 | |
| | | ATCC 33655 | |
| | | ATCC 700326 | |
| *V. diazotrophicus* | | ATCC 33466 T | |
| *V. navarrensis* | | ATCC 51183 T | |
| *V. metschnikovii* | | ATCC 700040 T | |
| *V. cincinnatiensis* | | ATCC 35912 T | |
| *V. ordalii* | | NCIMB 2167 | |
| *Listonella anguillarum* | | NCIMB 6 | |
| *V. hollisae* | | ATCC 33564 T | |
| *V. alginolyticus* | | IFO 15630 T | |
| *V. campbellii* | | IFO 15631 T | |
| *V. carchariae* | | IFO 15632 T | |
| *V. harveyi* | | IFO 15634 T | |
| *V. nereis* | | IFO 15637 T | |
| *V. parahaemolyticus* | | IFO 12711 T | |
| *V. proteolyticus* | | IFO 13287 T | |
| *V. tubiashii* | | IFO 15644 T | |
| | | Strains isolated from food | 5 strains |
| Total 85 strains | | | |

Specifically, after the test strains shown in Table 1 above were subjected to enrichment culture using brain heart infusion media supplemented with 2% NaCl, chromosomal DNA was extracted using a PUREGENE DNA Isolation Kit (Gentra SYSTEMS). Using the extracted DNA as a template, a *gyrB* gene fragment with a length of approximately 900 bp (a region corresponding to positions 331 to 1212 of the nucleotide sequence or positions 111 to 404 of the amino acid sequence of *Escherichia coli* strain K-12) was amplified by PCR using *gyrB* universal amplification primers UP-1E (5'-caggaaacagctatgaccaygsnggnggnaarttyra-3') and AprU (5'-tgtaaaacgacggccagtgcnggrtcyttytcytgrca-3'). Furthermore, similarly, an *rpoD* gene fragment with a length of approximately 800 bp (a region corresponding to positions 334 to 1125 of the nucleotide sequence and positions 112 to 376 of the amino acid sequence of *Escherichia coli* strain K-12) was amplified by PCR using *rpoD* universal amplification primers 70F-M13 (5'-caggaaacagctatgaccyatgmgngaratgggnacngt-3') and 70R-M13(5'-tgtaaaacgacggccagtngcytcnaccatytcyttytt-3'). Furthermore, a *recA* gene fragment with a length of 648 bp (a region corresponding to positions 172 to 819 of the nucleotide sequence and positions 58 to 273 of the amino acid sequence of *Escherichia coli* strain K-12) was amplified by PCR using *recA* gene universal amplification primers recUF and recUR (recUF: caggaaacagctatgaccatggaygtngaracnat and recUR: tgtaaaacgacggccagttanswrtaccangcncc). Amplification reaction was conducted using thermostable DNA polymerase (AmpliTaq Gold: produced by Applied Biosystems) and a GENE MATE thermal cycler (ISC BioExpress). 50 µl of a reaction solution was prepared to contain 1 µg of DNA, 50 mM KCl, 10 mM Tris-HCl (pH8.3), 1.5 mM MgCl₂, 0.01% gelatine, dNTP (0.2 mM each), 2.5 U of AmpliTaq Gold, and primers (1 µM each). Reaction was conducted under conditions of activation with AmpliTaq Gold (95 °C for 10 minutes), 40 cycles of 1 minute of reaction at 94 °C, 1 minute of annealing (56°C for *gyrB* and *rpoD*; 54°C for *recA*), and 2 minutes of reaction at 72°C, and then 10 minutes of elongation reaction at 72°C. The obtained PCR product was subjected to electrophoresis (0.5xTAE and 100 V for 30 minutes) using 1% agarose gel (SeaPlaque GTG agarose: produced by BioWhittaker Molecular Applications), and then stained with ethidium bromide for 10 minutes. Amplified products were confirmed to be present under UV irradiation, excised from the gel, and then purified using a Wizard PCR Preps DNA Purification System (produced by Promega), thereby preparing templates for sequence reaction. Sequence reaction was conducted using as primers M13R sequence (5'-CAggAAACAgCTATgACC-3') and M13-21 sequence (5'-TgTAAAACgACggCCAgT-3') previously added to the universal primers, an ABI PRISM BigDye Terminator Cycle Sequencing Ready Reaction Kit (produced by Applied Biosystems), and a GENE MATE thermal cycler (ISC BioExpress). A reaction solution was prepared to have a final volume of 20 µl by mixing 20 ng of DNA, 3.2 pmol of primers, and 8 µl of BigDye Terminator Ready Reaction Mix. Cycle sequence reaction was conducted by heating at 92°C for 10 minutes, and then conducting 25 cycles of reaction (96°C for 10 seconds, 50°C for 5 seconds, and 60°C for 4 minutes). For nucleotide sequence analysis, ABI PRISM 310 GENETIC ANALYZER (produced by Applied Biosystems) was used. Upon phylogenetic analysis using the obtained nucleotide sequences, to more precisely understand closely related species of *Vibrio vulnificus*, analysis was conducted after linking partial sequences of *gyrB, rpoD*, and *recA* genes. Specifically, after linking the above 3 gene sequences, multiple alignment analysis was conducted using a Clustal W computer program. Subsequently, through the use of a PHYLIP computer program package, a phylogenetic tree was produced by the neighbor-joining method (Mol. Biol. Evol. Vol. 4, No. 4, 406-425) based on the genetic distance calculated using Kimura's 2-parameter model (J. Mol. Evol. (1980) Vol. 16, No.2, pp. 111-20).

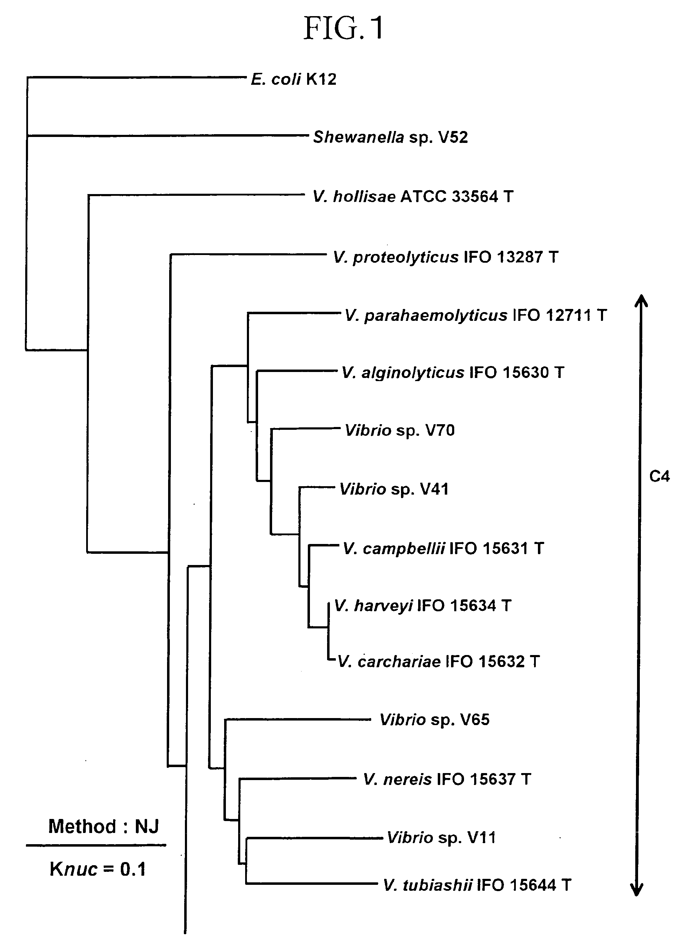

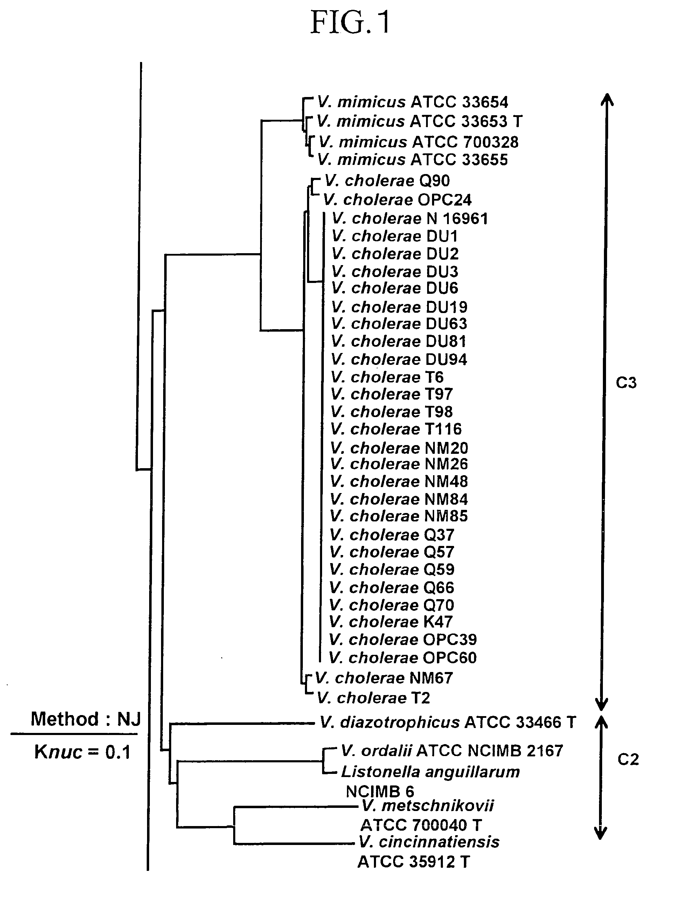

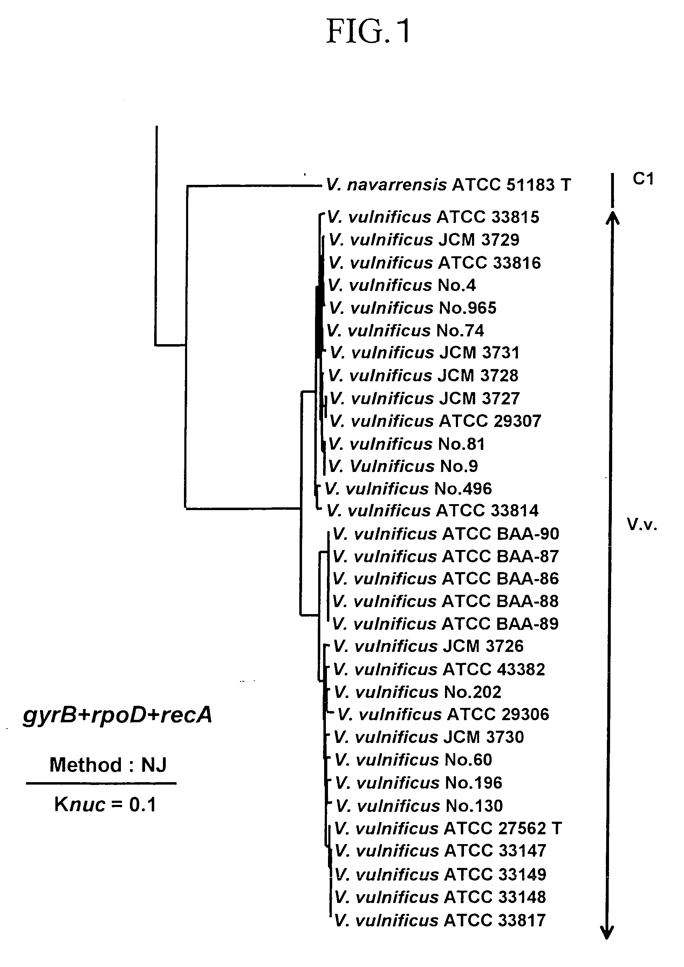

As a result, it was shown that all strains of *Vibrio vulnificus* subjected to analysis compose an independent mono phylem among strains of the genus *Vibrio* (Fig. 1). Specifically, it was suggested that bacterial groups belonging to this phylem should be determined to be *Vibrio vulnificus*.

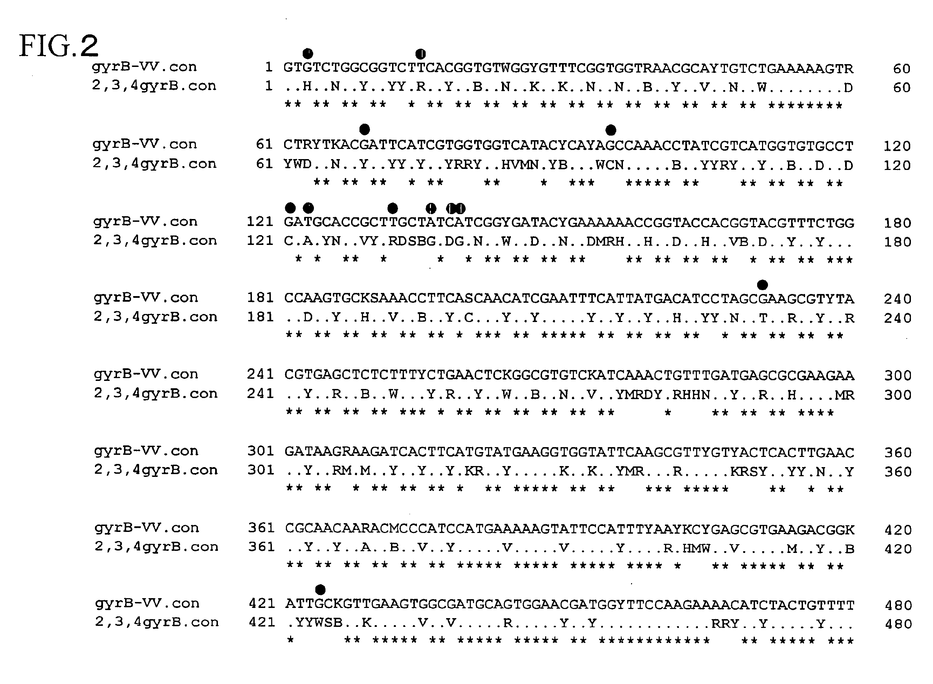

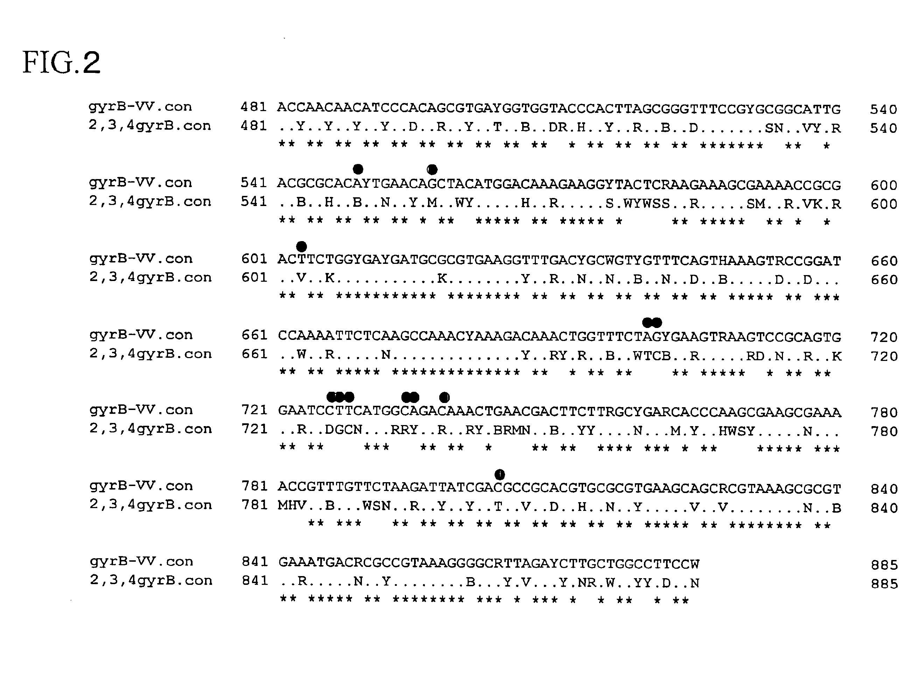

Hence, to establish a genetic method that can detect only a *Vibrio vulnificus* bacterial group, first differences in nucleotide sequences among closely related species were revealed. That is, nucleotide positions that are conserved within the *Vibrio vulnificus* group but differ from other bacteria of the genus *Vibrio* were identified. Specifically, consensus sequence of the phylogenetic group to Which *Vibrio vulnificus* belongs were found and compared with consensus sequence of C1 to C3 cluster in Fig. 1 determined to be related to this phylem based on the results of phylogenetic analysis. Thus, a phylogenetically-specific information map was produced (Figs. 2, 3, and 4). According to Figs. 2 to 4, it was shown that in the case of *gyrB* gene, positions 3, 15, 69, 95, 121, 123, 132, 136, 138, 139, 231, 424, 549, 557, 603, 700, 701, 726, 727, 728, 734, 735, 738, and 804 of SEQ ID NO: 1; in the case of *rpoD* gene, positions 21, 33, 60, 120, 126, 235, 260, 312, 315, 340, 354, 396, 414, 459, 462, 570, 686, 689, 732, 750, and 756 of SEQ ID NO: 2; and in the case of *recA* gene, positions 9, 81, 138, 153, 172, 180, 208, 228, 237, 288, and 540 of SEQ ID NO: 3 in the sequence listing are specific to the phylem to which *Vibrio vulnificus* belongs. The use of sequences specific to this phylem containing these characteristic nucleotides enables the design of probes having high specificity and gene amplification primers having high specificity and excellent amplification efficiency. For example, it becomes possible to design gene amplification primers using 15 or more continuous nucleotides (containing nucleotides differing from those of closely related species so as to always contain positions differing in terms of nucleotides from those of the closely related species) of nucleotide sequences of the *gyrB, rpoD*, and *recA* genes, preferably 20 or more nucleotides, and further preferably 20 or more nucleotides and 40 or fewer nucleotides of continuous nucleotide sequences of the *gyrB, rpoD*, and *recA* genes. Similarly, it also becomes possible to design probes using 15 or more continuous nucleotides (containing nucleotides differing from those of closely related species so as to always contain positions differing in terms of nucleotides from those of the closely related species) of nucleotide sequences of the *gyrB, rpoD,* and *recA* genes, preferably 20 or more nucleotides, and further preferably 20 or more nucleotides and 100 or fewer nucleotides of continuous nucleotide sequences of the *gyrB, rpoD,* and *recA* genes. Furthermore, to produce the primers and the probes, regions containing the above different nucleotides at high frequencies are preferably used, such as: in the case of the *gyrB* gene, a region containing 2 or more positions of any of positions 121, 123, 132, 136, 138, and 139, a region containing positions 549 and 557, a region containing positions 700 and 701, and a region containing 2 or more positions of any of positions 726, 727, 728, 734, 735, and 738; in the case of the *rpoD* gene, a region containing positions 21 and 33, a region containing positions 120 and 126, a region containing positions 312 and 315, a region containing positions 340 and 354, a region containing positions 396 and 414, a region containing positions 459 and 462, a region containing positions 686 and 689, and a region containing 2 or more positions of any of positions 732, 750, and 756; and in the case of *recA* gene, a region containing positions 138 and 153, a region containing positions 172 and 180, a region containing 2 or more positions of any of positions 208, 228, and 237, and a region containing positions 228 and 237.

Strands or complementary strands thereof comprising these regions can be used as gene amplification primers. In addition, other sequences such as an adapter sequence or a tag sequence can be included therein, if necessary.

In the case of the *gyrB*, an example of a preferred primer is: a strand (nucleic acid) comprising a sequence of 5'-gtgtctggcggtctt-3' (claim 10, SEQ ID NO: 4), 5'-gatgcaccgcttgctatcatc-3' (claim 11, SEQ ID NO: 5), 5'-tgcggacttyacttcrct-3' (claim 12, SEQ ID NO: 6), 5'-gtccatgtagctgttcart-3' (claim 13, SEQ ID NO: 7), 5'-ttgtctgccatgaaggattcc-3' (claim 14, SEQ ID NO: 8), or a complementary strand thereof; or a strand (nucleic acid) containing any one sequence of these sequences or a complementary strand (nucleic acid) thereof.

In the case of the *rpoD*, an example of a preferred primer is: a strand (nucleic acid) comprising a sequence of 5'-aatcgacatcgctaamcga-3' (claim 28, SEQ ID NO: 9), 5'-gttcgacaaagtacaagcg-3' (claim 29, SEQ ID NO: 10), 5'-tcaagcagygattcagag-3' (claim 30, SEQ ID NO: 11), 5'-aatggcgctagagaag-3' (claim 31, SEQ ID NO: 12), 5'-cktraatgaacatggtcga-3' (claim 32, SEQ ID NO: 13), 5'-gaactgatgctcgatgtgttt-3' (claim 33, SEQ ID NO: 14), 5'-aatgtcttcttcgtgmagyt-3' (claim 34, SEQ ID NO: 15), 5'-ttgatgttgytyactgaaagc-3' (claim 35, SEQ ID NO: 16), or a complementary strand thereof; or a strand (nucleic acid) containing any one sequence of these sequences or a complementary strand (nucleic acid) thereof.

In the case of the *RecA,* an example of a preferred primer is: a strand (nucleic acid) comprising a sequence of 5'-cctgtgtatgcgaagaarctt-3' (claim 44, SEQ ID NO: 17), 5'-tatcgaccarttrttggta-3' (claim 45, SEQ ID NO: 18), 5'-aagmgcatcacagatttccaa-3' (claim 46, SEQ ID NO: 19), 5'-tcaaccgcmcctgagcgagca-3' (claim 47, SEQ ID NO: 20), or a complementary strand thereof; or a strand (nucleic acid) containing any one sequence of these sequences or a complementary strand (nucleic acid) thereof.

Furthermore, in the case of a primer, the 3'-terminus is preferably a nucleotide specific to *Vibrio vulnificus*. The present invention encompasses a kit for detecting, quantifying, or identifying *Vibrio vulnificus* using these primers and probes in combination with other reagents.

A gene amplification method using the primers of the present invention is not limited to the PCR method. The primers can be used in a specific amplification method that is based on the specificity of primers or a method for specifically inhibiting amplification. Furthermore, the primers can also be similarly used in a quantification and amplification reaction using specific amplification primers and a labeled specific probe in combination. Furthermore, the probe of the present invention can also be used alone. A method for using the probe is not limited to one involving the solid phase or the liquid phase.

The primers and the probes can also be utilized for carrying out real-time PCR using a reagent such as cyber green for detecting amplified double-stranded DNA or real-time PCR to which FRET or the like is applied.

This specification includes part or all of the contents as disclosed in the specification or drawings of Japanese Patent Application No. 2001-306868, which is a priority document of the present application.

### BRIEF DESCRIPTION OF THE DRAWINGS

Fig. 1 shows the result of phylogenetic analysis after linking partial sequences of the *gyrB, rpoD*, and *recA* genes. This is a phylogenetic tree produced by the neighbor-joining method, showing that *Vibrio vulnificus* (denoted as V.v. in the figure) belongs to a mono phylem differing from those of other bacteria belonging to the genus *Vibrio.* Furthermore, cluster other than that of *Vibrio vulnificus* are classified into C1 to C4. As gene data of *E. coli* K12 strain and *V. cholerae* N 16961 strain, accession numbers NC00913 and NC002505 in the GenBank database are used, respectively. In addition, figures are arranged from the top to the bottom according to page number.
Fig. 2 shows the result of determination of and comparison between consensus sequence (upper case) of the *gyrB* gene of the cluster shown in Fig. 1 to which *Vibrio vulnificus* belongs and consensus sequence (lower case) of the *gyrB* gene of related clusters (C1, 2, and 3 in Fig. 1). It is shown that sites denoted with ● are nucleotides specific to *Vibrio vulnificus.* It is shown that D = A or G or T, H = A or C or T, V = A or C or G, R = A or G, Y = C or T, K = G or T, M = A or C, S = G or C, W = A or T, and N = A or G or T or C.

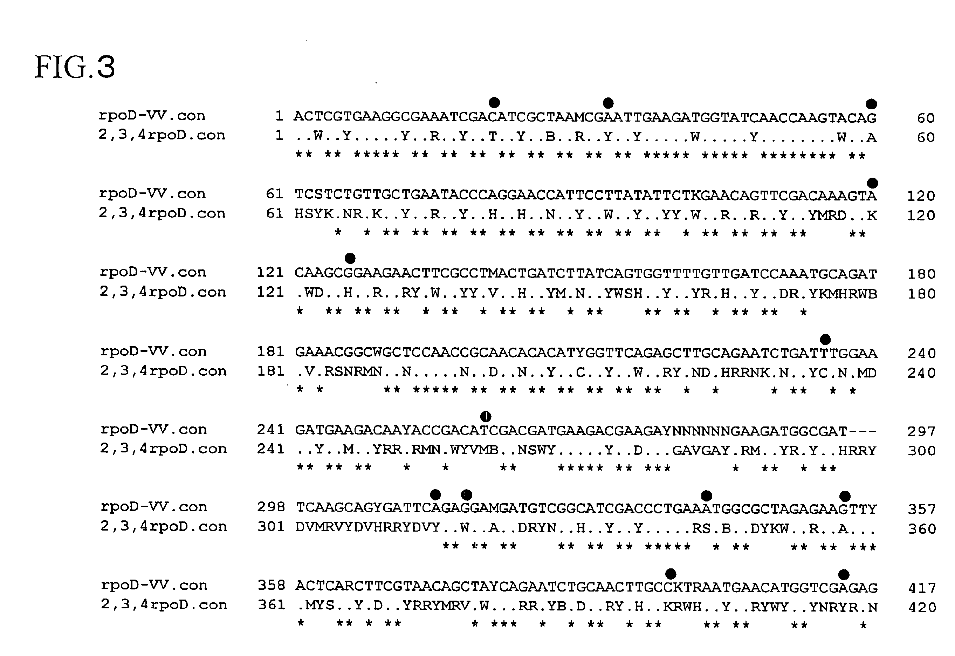

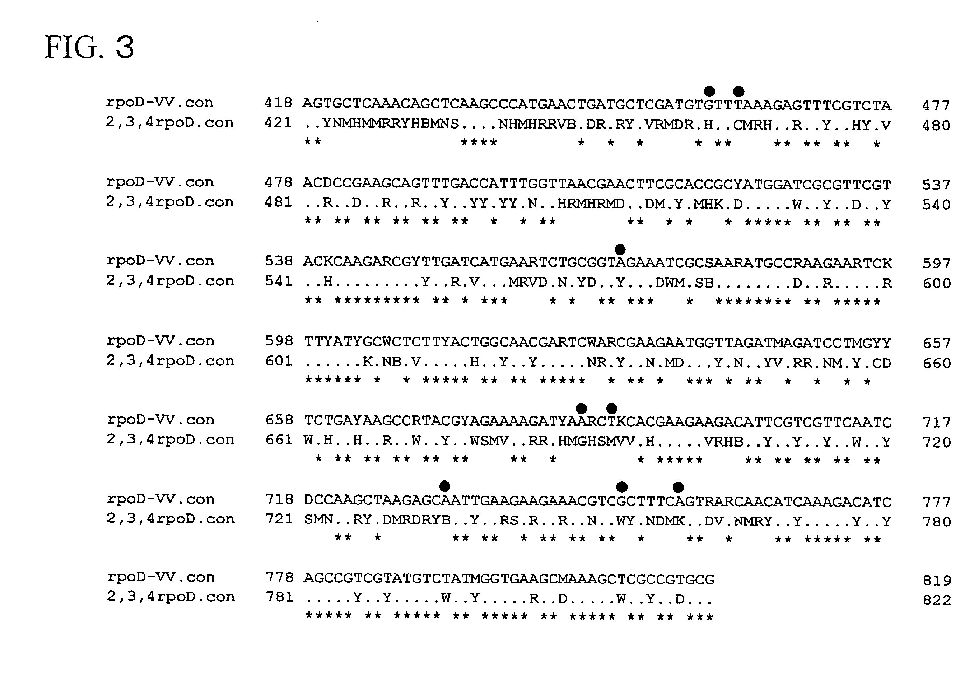

Fig. 3 shows the result of determination of and comparison between consensus sequence (upper case) of the *rpoD* gene of the cluster shown in Fig. 1 to which *Vibrio vulnificus* belongs and consensus sequence (lower case) of the *rpoD* gene of the related clusters (C1, 2, and 3 in Fig. 1). It is shown that sites denoted with ● are nucleotides specific to *Vibrio vulnificus*. It is shown that D = A or G or T, H = A or C or T, V = A or C or G, R = A or G, Y = C or T, K = G or T, M = A or C, S = G or C, W = A or T, and N = A or G or T or C.

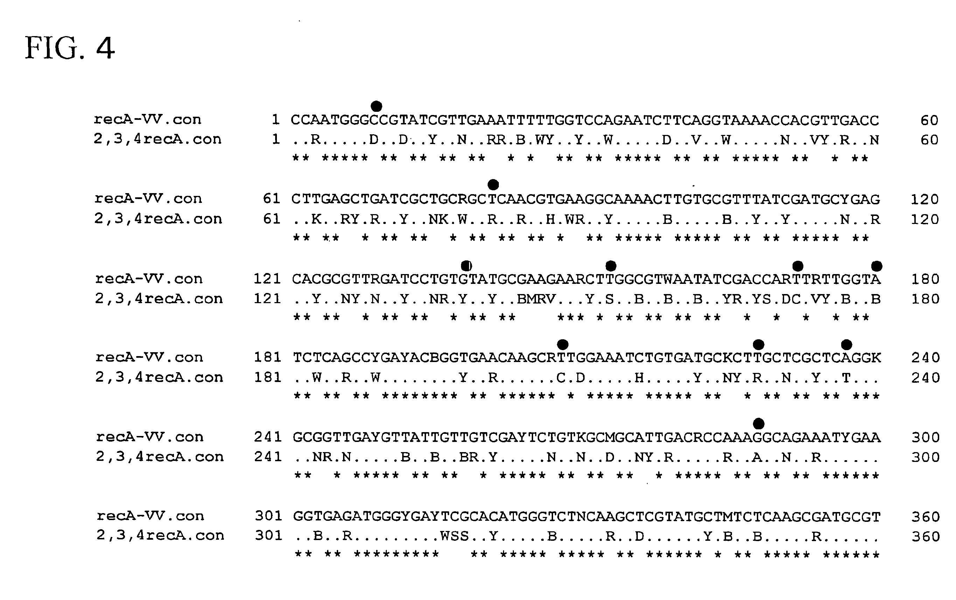

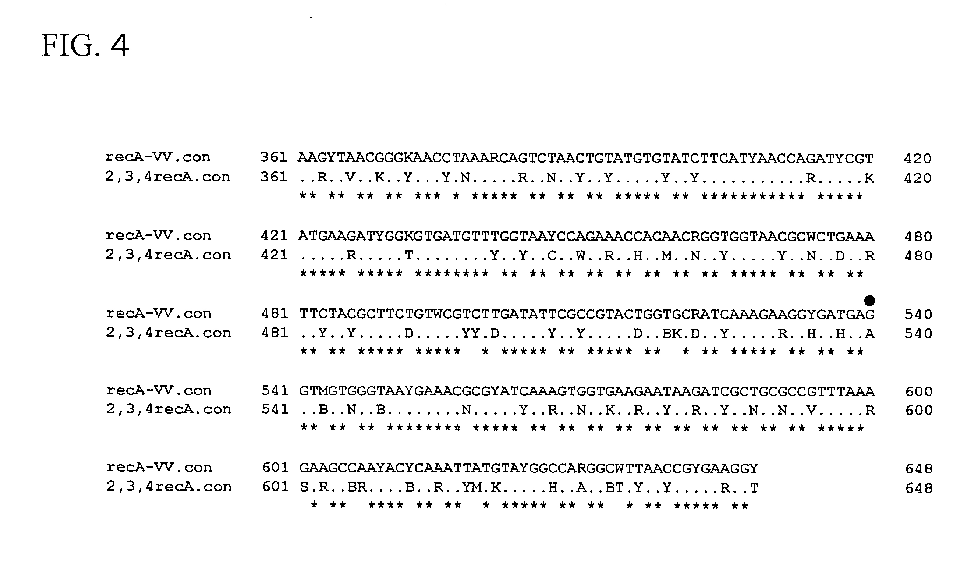

Fig. 4 shows the result of determination of and comparison between consensus sequence (upper case) of the *recA* gene of the cluster shown in Fig. 1 to which *Vibrio vulnificus* belongs and consensus sequence (lower case) of the *recA* gene of the related clusters (C1, 2, and 3 in Fig. 1). It is shown that sites denoted with ● are nucleotides specific to *Vibrio vulnificus*. It is shown that D = A or G or T, H = A or C or T, V = A or C or G, R = A or G, Y = C or T, K = G or T, M = A or C, S = G or C, W = A or T, and N = A or G or T or C.

### BEST MODE OF CARRYING OUT THE INVENTION

The present invention will be hereafter described in detail by referring to examples, but the invention is not limited by these examples.

### [Example 1]

An example of using primers for gene amplification (shown in Table 2) designed and obtained according to the present invention using regions specific to *Vibrio vulnificus* is shown.

**Table 2.**

| Primers for specifically detecting *Vibrio vulnificus* | | | | | |
|---|---|---|---|---|---|
| Target gene | Primer | Sequence | Length | Position^{a)} | Direction |
| *gyrB* | VF1 VR1 | 5'-gatgcaccgcttgctatcatc-3' 5'-ttgtctgccatgaaggattcc-3' | 21 21 | 121-141 740-720 | Sense Antisense |
| *rpoD* | VrF2 VrR2 | 5'-gaactgatgctcgatgtgttt-3' 5'-ttgatgttgytyactgaaagc-3' | 21 21 | 442-462 770-750 | Sense Antisense |
| *recA* | VVrecF2 VVrecR2 | 5'-cctgtgtatgcgaagaarctt-3' 5'-tcaaccgcmcctgagcgagca-3' | 21 21 | 133-153 248-228 | Sense Antisense |

| | | | | | |
|---|---|---|---|---|---|
| a): denotes positions from the 5' terminus in the nucleotide sequences represented by SEQ ID NOS: 1 to 3. | | | | | |

In addition, primers described in claims 11, 14, 33, 35, 44, and 47 correspond to VF1, VR1, VrF2, VrR2, VVrecF2, and VVrecR2 in Table 2 and are represented by SEQ ID NO: 5, SEQ ID NO: 8, SEQ ID NO: 14, SEQ ID NO: 16, SEQ ID NO: 17, and SEQ ID NO: 20, respectively.

PCR was carried out using chromosomal DNAs as templates extracted from test strains. Amplification reaction was conducted using thermostable DNA polymerase (AmpliTaq Gold: produced by Applied Biosystems) and a GENE MATE thermal cycler (ISC BioExpress). A reaction solution was prepared to have a final volume of 20 µl and to contain 0.1 µg of DNA, 50 mM KCl, 10 mM Tris-HCl (pH8.3), 1.5 mM MgCl₂, 0.01% gelatine, dNTP (0.2 mM each), 2.5 U of AmpliTaq Gold, and primers (for the concentration, see Table 3). Reaction was conducted under conditions of activation with AmpliTaq Gold (95°C for 10 minutes), 35 cycles of 94°C for 1 minute, 1 minute of annealing (65°C for the *gyrB* gene, 63°C for the *rpoD* gene, and 65°C for the *recA* gene), and 72°C for 1 minute, finally followed by 10 minutes of elongation reaction at 72°C. Primer combinations and the amplification reaction conditions are shown in Table 3.

**Table 3.**

| PCR conditions for primers for specifically detecting *Vibrio vulnificus* | | | | | | |
|---|---|---|---|---|---|---|
| Target gene | Sense primer | Sense primer | Amplified product (bp) | PCR conditions | | |
| | | | | Annealing temperature (°C) | Number of cycles | Primer concentration (µM) |
| *gyrB* | VF1 | VR1 | 620 | 65 | 35 | 0.1 |
| *rpoD* | VrF2 | VrR2 | 329 | 55 | 35 | 0.1 |
| *recA* | VVrecF2 | VVrecR2 | 116 | 60 | 35 | 0.1 |

5 µl of the reaction solution following amplification was subjected to electrophoresis (0.5xTAE and 100V for 30 minutes) using 1% agarose gel (agarose S: produced by NIPPON GENE CO., LTD.) and then staining with ethidium bromide. The presence or the absence of amplified genes was confirmed under UV irradiation. DNAs derived from the strains shown in Table 1 were screened for by PCR using combinations of VF1 and VR1 primers targeting the *gyrB* gene, VrF2 and VrR2 primers targeting the *rpoD* gene, and VVrecF2 and VVrecR2 primers targeting the *recA* gene. The results are shown in Table 4. Amplified products were confirmed for only the DNAs derived from the strains determined to belong to *Vibrio vulnificus* as shown in Fig. 1, revealing that for all combinations, only *Vibrio vulnificus* can be detected.

**Table 4**

| Results of PCR using primers for specifically detecting *V. vulnificus* | | | | | | |
|---|---|---|---|---|---|---|
| | | | | **PCR** | | |
| **No.** | **Cluster** | **Name** | **Strain name** | **VF1&** | **VrF2&** | **VVrecF2&** |
| | | | | **VR1** | **VrR2** | **VVrecR2** |
| 1 | V.v | *V. vulnificus* | ATCC 27562 T | + | + | + |
| 2 | V.v | *V. vulnificus* | ATCC 29306 | + | + | + |
| 3 | V.v | *V. vulnificus* | ATCC 29307 | + | + | + |
| 4 | V.v | *V. vulnificus* | ATCC 33147 | + | + | + |
| 5 | V.v | *V. vulnificus* | ATCC 33148 | + | + | + |
| 6 | V.v | *V. vulnificus* | ATCC 33149 | + | + | + |
| 7 | V.v | *V. vulnificus* | ATCC 33814 | + | + | + |
| 8 | V.v | *V. vulnificus* | ATCC 33815 | + | + | + |
| 9 | V.v | *V. vulnifcus* | ATCC 33816 | + | + | + |
| 10 | V.v | *V. vulnificus* | ATCC 33817 | + | + | + |
| 11 | V.v | *V. vulnificus* | ATCC 43382 | + | + | + |
| 12 | V.v | *V. vulnificus* | ATCC BAA-86 | + | + | + |
| 13 | V.v | *V. vulnificus* | ATCC BAA-87 | + | + | + |
| 14 | V.v | *V. vulnificus* | ATCC BAA-88 | + | + | + |
| 15 | V.v | *V. vulnificus* | ATCC BAA-89 | + | + | + |
| 16 | V.v | *V. vulnificus* | ATCC BAA-90 | + | + | + |
| 17 | V.v | *V. vulnificus* | JCM 3726 | + | + | + |
| 18 | V.v | *V. vulnificus* | JCM 3727 | + | + | + |
| 19 | V.v | *V. vulnificus* | JCM 3728 | + | + | + |
| 20 | V.v | *V. vulnificus* | JCM 3729 | + | + | + |
| 21 | V.v | *V. vulnificus* | JCM 3730 | + | + | + |
| 22 | V.v | *V. vulnificus* | JCM 3731 | + | + | + |
| 23 | V.v | *V. vulnificus* | No.4 | + | + | + |
| 24 | V.v | *V. vulnificus* | No.9 | + | + | + |
| 25 | V.v | *V. vulnificus* | No.60 | + | + | + |
| 26 | V.v | *V. vulnificus* | No.74 | + | + | + |
| 27 | V.v | *V. vulnificus* | No.81 | + | + | + |
| 28 | V.v | *V. vulnificus* | No.130 | + | + | + |
| 29 | V.v | *V. vulnificus* | No.196 | + | + | + |
| 30 | V.v | *V. vulnificus* | No.202 | + | + | + |
| 31 | V.v | *V. vulnificus* | No.496 | + | + | + |
| 32 | V.v | *V. vulnificus* | No.965 | + | + | + |
| 33 | C3 | *V. cholerae* | DU1 | - | - | - |
| 34 | C3 | *V. cholerae* | DU2 | - | - | - |
| 35 | C3 | *V. cholerae* | DU3 | - | - | - |
| 36 | C3 | *V. cholerae* | DU6 | - | - | - |
| 37 | C3 | *V. cholerae* | DU19 | - | - | - |
| 38 | C3 | *V. cholerae* | DU63 | - | - | - |
| 39 | C3 | *V. cholerae* | DU81 | - | - | - |
| 40 | C3 | *V. cholerae* | DU94 | - | - | - |
| 41 | C3 | *V. cholerae* | T2 | - | - | - |
| 42 | C3 | *V. cholerae* | T6 | - | - | - |
| 43 | C3 | *V. cholerae* | T97 | - | - | - |
| 44 | C3 | *V. cholerae* | T98 | - | - | - |
| 45 | C3 | *V. cholerae* | T116 | - | - | - |
| 46 | C3 | *V. cholerae* | NM20 | - | - | - |
| 47 | C3 | *V. cholerae* | NM26 | - | - | - |
| 48 | C3 | *V. cholerae* | NM48 | - | - | - |
| 49 | C3 | *V. cholerae* | NM67 | - | - | - |
| 50 | C3 | *V. cholerae* | NM84 | - | - | - |
| 51 | C3 | *V cholerae* | NM85 | - | - | - |
| 52 | C3 | *V. cholerae* | Q37 | - | - | - |
| 53 | C3 | *V. cholerae* | Q57 | - | - | - |
| 54 | C3 | *V. cholerae* | Q59 | - | - | - |
| 55 | C3 | *V. cholerae* | Q66 | - | - | - |
| 56 | C3 | *V. cholerae* | Q70 | - | - | - |
| 57 | C3 | *V. cholerae* | Q90 | - | - | - |
| 58 | C3 | *V. cholerae* | K47 | - | - | - |
| 59 | C3 | *V. cholerae* | OPC24 | - | - | - |
| 60 | C3 | *V. cholerae* | OPC39 | - | - | - |
| 61 | C3 | *V. cholerae* | OPC60 | - | - | - |
| 62 | C3 | *V. mimicus* | ATCC 33563 T | - | - | - |
| 63 | C3 | *V. mimicus* | ATCC 33654 | - | - | - |
| 64 | C3 | *V. mimicus* | ATCC 33655 | - | - | - |
| 65 | C3 | *V. mimicus* | ATCC 7000326 | - | - | - |
| 66 | C2 | *V. diazotrophicus* | ATCC 33466 T | - | - | - |
| 67 | C1 | *V. navarrensis* | ATCC 51183 T | - | - | - |
| 68 | C2 | *V. metschnikovii* | ATCC 700040 T | - | - | - |
| 69 | C2 | *V. cincinnatiensis* | ATCC 35912 T | - | - | - |
| 70 | C2 | *V ordalii* | NCIMB 2167 | - | - | - |
| 71 | C2 | *istonella anguillarun* | NCIMB 6 | - | - | - |
| 72 | C4 | *V. alginolyticus* | IFO 15630 T | - | - | - |
| 73 | C4 | *V. campbellii* | IFO 15631 T | - | - | - |
| 74 | C4 | *V. carchariae* | IFO 15632 T | - | - | - |
| 75 | C4 | *V. harveyi* | IFO 15634 T | - | - | - |
| 76 | C4 | *V nereis* | IFO 15637 T | - | - | - |
| 77 | C4 | *V. parahaemolyticus* | IFO 12711 T | - | - | - |
| 78 | | *V. proteolyticus* | IFO 13287 T | - | - | - |
| 79 | C4 | *V. tubiashii* | IFO 15644 T | - | - | - |
| 80 | C4 | *Vibrio* sp. | V11 | - | - | - |
| 81 | C4 | *Vibrio* sp. | V41 | - | - | - |
| 82 | | *Shewanella* sp. | V52 | - | - | - |
| 83 | C4 | *Vibrio* sp. | V65 | - | - | - |
| 84 | C4 | *Vibrio* sp. | V70 | - | - | - |
| 85 | | *V. hollisae* | ATCC 33564 T | - | - | - |

All publications, patents and patent applications cited herein are incorporated herein by reference in their entirety.

### Industrial Applicability

The *gyrB, rpoD*, and *recA* gene primers and probes of the present invention have been designed based on the understanding of the phylogenetic relationship with *Vibrio vulnificus*. Thus, the primers and the probes have been studied for improving specificity and are excellent in terms of detection accuracy. Hence, the primers and the probes are advantageous when direct detection is carried out under circumstances where a bacterium is not isolated from foods, clinical specimens, or the like and related bacterial species are contaminated.
Sequence Listing Free Text
SEQ ID NOS: 4 to 20 are primers.

## Claims

1. A gene fragment represented by SEQ ID NO: 1, which contains one or more nucleotides at any of positions 3, 15, 69, 95, 121, 123, 132, 136, 138, 139, 231, 424, 549, 557, 603, 700, 701, 726, 727, 728, 734, 735, 738, and 804 in a gene (*gyrB*) encoding a DNA gyrase β subunit of SEQ ID NO: 1 in the sequence listing, said position being unique to *Vibrio vulnificus* bacterial group, where said gene fragment can be used for designing a specific gene amplification primer or a specific probe.

2. A gene amplification primer, which contains a strand or a complementary strand thereof containing 15 or more continuous nucleotides, where one or more nucleotides are nucleotides at any of positions 3, 15, 69, 95, 121, 123, 132, 136, 138, 139, 231, 424, 549, 557, 603, 700, 701, 726, 727, 728, 734, 735, 738, and 804 in the gene (*gyrB*) encoding the DNA gyrase β subunit of SEQ ID NO: 1 in the sequence listing, said positions being unique to the *Vibrio vulnificus* bacterial group.

3. The gene amplification primer of claim 2, wherein a region that contains, at a high frequency, a position(s) unique to *Vibrio vulnificus* as specified in claim 2 is used.

4. The gene amplification primer of claim 3, which contains a strand or a complementary strand thereof containing nucleotides at positions 3 and 15 of SEQ ID NO: 1 in the sequence listing.

5. The gene amplification primer of claim 3, which contains a strand or a complementary strand thereof containing 2 or more nucleotides at any of positions 121, 123, 132, 136, 138, and 139 of SEQ ID NO: 1 in the sequence listing.

6. The gene amplification primer of claim 3, which contains a strand or a complementary strand thereof containing nucleotides at positions 549 and 557 of SEQ ID NO: 1 in the sequence listing.

7. The gene amplification primer of claim 3, which contains a strand or a complementary strand thereof containing nucleotides at positions 700 and 701 of SEQ ID NO: 1 in the sequence listing.

8. The gene amplification primer of claim 3, which contains a strand or a complementary strand thereof containing 2 or more nucleotides at any of positions 726, 727, 728, 734, 735, and 738 of SEQ ID NO: 1 in the sequence listing.

9. The gene amplification primer of claim 2, wherein the 3' terminal nucleotide is a nucleotide at a position that is unique to *Vibrio vulnificus* as specified in claim 2.

10. The gene amplification primer of claim 2, which contains 5'-gtgtctggcggtctt-3' or a complementary strand corresponding thereto.

11. The gene amplification primer of claim 2, which contains 5'-gatgcaccgcttgctatcatc-3' or a complementary strand corresponding thereto.

12. The gene amplification primer of claim 2, which contains 5'-tgcggacttyacttcrct-3' or a complementary strand corresponding thereto.

13. The gene amplification primer of claim 2, which contains 5'-gtccatgtagctgttcart-3' or a complementary strand corresponding thereto.

14. The gene amplification primer of claim 2, which contains 5'-ttgtctgccatgaaggattcc-3' or a complementary strand corresponding thereto.

15. A probe for detecting, quantifying, or identifying *Vibrio vulnificus*, which contains 15 or more continuous nucleotides, where one or more nucleotides are nucleotides at any of positions 3, 15, 69, 95, 121, 123, 132, 136, 138, 139, 231, 424, 549, 557, 603, 700, 701, 726, 727, 728, 734, 735, 738, and 804 in the gene (*gyrB*) encoding the DNA gyrase β subunit of SEQ ID NO: 1 in the sequence listing, said positions being unique to the *Vibrio vulnificus* bacterial group.

16. A gene fragment represented by SEQ ID NO: 2, which contains one or more nucleotides at any of positions 21, 33, 60, 120, 126, 235, 260, 312, 315, 340, 354, 396, 414, 459, 462, 570, 686, 689, 732, 750, and 756 in a gene (*rpoD*) encoding an RNA polymerase σ 70 factor of SEQ ID NO: 2 in the sequence listing, said positions being unique to the *Vibrio vulnificus* bacterial group, where such gene fragment can be used for designing a specific gene amplification primer or a specific probe.

17. A gene amplification primer, which contains a strand or a complementary strand thereof containing 15 or more continuous nucleotides, where one or more nucleotides are nucleotides at any of positions 21, 33, 60, 120, 126, 235, 260, 312, 315, 340, 354, 396, 414, 459, 462, 570, 686, 689, 732, 750, and 756 in the gene (*rpoD*) encoding the RNA polymerase σ 70 factor of SEQ ID NO: 2 in the sequence listing, said positions being unique to the *Vibrio vulnificus* bacterial group.

18. The gene amplification primer of claim 17, wherein a region that contains, at a high frequency, a position(s) unique to *Vibrio vulnificus* as specified in claim 17 is used.

19. The gene amplification primer of claim 18, which contains a strand or a complementary strand thereof containing nucleotides at positions 21 and 33 of SEQ ID NO: 2 in the sequence listing.

20. The gene amplification primer of claim 18, which contains a strand or a complementary strand thereof containing nucleotides at positions 120 and 126 of SEQ ID NO: 2 in the sequence listing.

21. The gene amplification primer of claim 18, which contains a strand or a complementary strand thereof containing nucleotides at positions 312 and 315 of SEQ ID NO: 2 in the sequence listing.

22. The gene amplification primer of claim 18, which contains a strand or a complementary strand thereof containing nucleotides at positions 340 and 354 of SEQ ID NO: 2 in the sequence listing.

23. The gene amplification primer of claim 18, which contains a strand or a complementary strand thereof containing nucleotides at positions 396 and 414 of SEQ ID NO: 2 in the sequence listing.

24. The gene amplification primer of claim 18, which contains a strand or a complementary strand thereof containing nucleotides at positions 459 and 462 of SEQ ID NO: 2 in the sequence listing.

25. The gene amplification primer of claim 18, which contains a strand or a complementary strand thereof containing nucleotides at positions 686 and 689 of SEQ ID NO: 2 in the sequence listing.

26. The gene amplification primer of claim 18, which contains a strand or a complementary strand thereof containing 2 or more nucleotides at any of positions 732, 750, and 756 of SEQ ID NO: 2 in the sequence listing.

27. The gene amplification primer of claim 17, wherein the 3' terminal nucleotide is a nucleotide at a position that is unique to *Vibrio vulnificus* as specified in claim 17.

28. The gene amplification primer of claim 17, which contains 5'-aatcgacatcgctaamcga-3' or a complementary strand corresponding thereto.

29. The gene amplification primer of claim 17, which contains 5'-gttcgacaaagtacaagcg-3' or a complementary strand corresponding thereto.

30. The gene amplification primer of claim 17, which contains 5'-tcaagcagygattcagag-3' or a complementary strand corresponding thereto.

31. The gene amplification primer of claim 17, which contains 5'-aatggcgctagagaag-3' or a complementary strand corresponding thereto.

32. The gene amplification primer of claim 17, which contains 5'-cktraatgaacatggtcga-3' or a complementary strand corresponding thereto.

33. The gene amplification primer of claim 17, which contains 5'-gaactgatgctcgatgtgttt-3' or a complementary strand corresponding thereto.

34. The gene amplification primer of claim 17, which contains 5'-aatgtcttcttcgtgmagyt-3' or a complementary strand corresponding thereto.

35. The gene amplification primer of claim 17, which contains 5'-ttgatgttgytyactgaaagc-3' or a complementary strand corresponding thereto.

36. A probe for detecting, quantifying, or identifying *Vibrio vulnificus,* which contains 15 or more continuous nucleotides, where one or more nucleotides are nucleotides at any of positions 21, 33, 60, 120, 126, 235, 260, 312, 315, 340, 354, 396, 414, 459, 462, 570, 686, 689, 732, 750, and 756 in the gene (*rpoD*) encoding the RNA polymerase σ 70 factor of SEQ ID NO: 2 in the sequence listing, said positions being unique to the *Vibrio vulnificus* bacterial group.

37. A gene fragment represented by SEQ ID NO: 3, which contains one or more nucleotides at any of positions 9, 81, 138, 153, 172, 180, 208, 228, 237, 288, and 540 in a gene (*recA*) encoding RecA of SEQ ID NO: 3 in the sequence listing, said positions being unique to the *Vibrio vulnificus* bacterial group, where said gene fragment can be used for designing a specific gene amplification primer or a specific probe.

38. A gene amplification primer, which contains a strand or a complementary strand thereof containing 15 or more continuous nucleotides, where one or more nucleotides are nucleotides at any of positions 9, 81, 138, 153, 172, 180, 208, 228, 237, 288, and 540 in the gene (*recA*) encoding the RecA of SEQ ID NO: 3 in the sequence listing, said positions being unique to the *Vibrio vulnificus* bacterial group.

39. The gene amplification primer of claim 38, wherein a region that contains, at a high frequency, a position(s) unique to *Vibrio vulnificus* as specified in claim 38 is used.

40. The gene amplification primer of claim 39, which contains a strand or a complementary strand thereof containing nucleotides at positions 138 and 153 of SEQ ID NO: 3 in the sequence listing.

41. The gene amplification primer of claim 39, which contains a strand or a complementary strand thereof containing nucleotides at positions 172 and 180 of SEQ ID NO: 3 in the sequence listing.

42. The gene amplification primer of claim 39, which contains a strand or a complementary strand thereof containing 2 or more nucleotides at any of positions 208, 228, and 237 of SEQ ID NO: 3 in the sequence listing.

43. The gene amplification primer of claim 39, wherein the 3' terminal nucleotide is a nucleotide at a position that is unique to *Vibrio vulnificus* as specified in claim 39.

44. The gene amplification primer of claim 38, which contains 5'-cctgtgtatgcgaagaarctt-3' or a complementary strand corresponding thereto.

45. The gene amplification primer of claim 38, which contains 5'-tatcgaccarttrttggta-3' or a complementary strand corresponding thereto.

46. The gene amplification primer of claim 38, which contains 5'-aagmgcatcacagatttccaa-3' or a complementary strand corresponding thereto.

47. The gene amplification primer of claim 38, which contains 5'-tcaaccgcmcctgagcgagca-3' or a complementary strand corresponding thereto.

48. A probe for detecting, quantifying, or identifying *Vibrio vulnificus,* which contains 15 or more continuous nucleotides, where one or more nucleotides are nucleotides at any of positions 9, 81, 138, 153, 172, 180, 208, 228, 237, 288, and 540 in the gene (*recA*) encoding the RecA of SEQ ID NO: 3 in the sequence listing, said positions being unique to the *Vibrio vulnificus* bacterial group.

49. A method for detecting, quantifying, or identifying *Vibrio vulnificus,* wherein the primer of any one of claims 2 to 14, 17 to 35, and 38 to 47 is used.

50. A kit for detecting, quantifying, or identifying *Vibrio vulnificus*, wherein the primer of any one of claims 2 to 14, 17 to 35, and 38 to 47 is used.
